# NEUE EUROPÄISCHE PATENTSCHRIFT

(11) **EP 0 440 147 B2**
(45) Veröffentlichungstag und Bekanntmachung des Hinweises auf die Entscheidung über den Einspruch: **14.05.2014**
(45) Hinweis auf die Patenterteilung: 22.09.2004
(21) Anmeldenummer: 91101095.7
(22) Anmeldetag: 28.01.1991
(51) Int. Cl.: C12N 15/13, C12P 21/08, C12Q 1/68, G01N 33/53

(54) **Herstellung und Verwendung von Genbanken menschlicher Antikörper ("Human-Antikörper-Bibliotheken")**
Preparation and use of a human antibody gene bank (human antibody libraries)
Préparation et utilisation de banques de gènes d'anticorps humains (bibliothèques d'anticorps humains)

(30) Priorität: 01.02.1990 DE 4002898; 09.02.1990 DE 4003881
(43) Veröffentlichungstag der Anmeldung: 07.08.1991
(60) Teilanmeldung: 04008412.1
(73) Patentinhaber: Siemens Healthcare Diagnostics Products GmbH, 35041 Marburg (DE)
(72) Erfinder: Little, Melvyn, Dr., W-6903 Neckargemünd (DE); Breitling, Frank Berthold, W-6908 Wiesoch (DE); Seehaus, Thomas, Dr., W-6148 Heppenheim (DE); Dübel, Stefan, W-6900 Heidelberg (DE); Klewinghaus, Iris, W-6800 Mannheim (DE)

(56) Entgegenhaltungen:
- EP-A1- 0 368 684
- EP-B1- 0 440 147
- WO-A1-90/14424
- WO-A1-90/14430
- WO-A1-90/14430
- WO-A1-91/10737
- NATURE. Bd. 349, 24. Januar 1991, LONDON GB Seiten 293 - 299; G. WINTER & C. MILSTEIN: 'Man-made antibodies' & XP000572614
- R. ORLANDI ET AL.: 'Cloning immunoglobulin variable domains for expression by the polymerase chain reaction' PROC. NAT. ACAD. SCI. USA Bd. 86, Mai 1989, Seiten 3833 - 3837
- Essential Immunology, 1988. Sixth Edition, Ed: Roit
- Immunology, 1989. Second Edition, Ed: Roit, Brostoff and Male
- LARRICK ET AL.: 'Polymerase Chain Reaction Using mixed Primers: Cloning of Human Monoclonal Antibody variable Region Genes from Single Hybridoma Cells' BIO/TECHNOLOGY Bd. 7, September 1989, Seiten 934 - 938
- SASTRY ET AL.: 'Cloning of the immunological repertoire in Escherichia coli for generation of monoclonal catalytic antibodies: Construction of a heavy chain variable region-specific cDNA library' PROC. NATL. ACAD. SCI. USA Bd. 86, August 1989, Seiten 5728 - 5732
- WARD ET AL.: 'Binding activities of a repertoire of single immunoglobulin variable domains secreted from Escherichia coli' LETTER TO NATURE Bd. 341, 12 Oktober 1989, Seiten 544 - 546
- LARRICK ET AL.: 'Rapid cloning of rearranged Immunoglobulin Genes from Human Hybridoma Cells using mixed Primers and the Polymerase Chain Reaction' BIOCHEMICAL AND BIOPHYSICAL RESEARCH COMMUNICATIONS Bd. 160, Nr. 3, 15 Mai 1989, Seiten 1250 - 1256
- S. LEVY ET AL.: 'A rapid method for cloning and sequencing variable-region genes of expressed immunoglobulins' GENE Bd. 54, 1987, Seiten 167 - 173
- Reply to Office Action dated 3 April 2001 during the prosecution of US6319690
- AMANN ET AL.: 'ATG vectors for regulated high-level expression of cloned genes in Escherichia coli' GENE Bd. 40, 1985, Seiten 183 - 190
- STRAUS ET AL.: 'Chicken triosephosphate isomerase complements an Escherichia coli deficiency' PROC. NATL. ACAD. SCI. USA Bd. 82, April 1985, Seiten 2014 - 2018
- HUSE ET AL.: 'Generation of a large combinatorial library of the Immunoglobulin Repertoire in Phage Lambda' SCIENCE Nr. 4935, 08 Dezember 1989, Seiten 1275 - 1281
- Advanced Immunology, 1987 Eds.: Male, Champion, Cooke
- LARRICK ET AL.: 'In Vitro Immunization in Hybridoma Technology', 1988, BORREBAECK Seiten 231 - 246
- CHIANG ET AL.: 'Direct cDNA Cloning of the Rearranged Immunoglobulin Variable Region' BIO TECHNIQUES Bd. 7, Nr. 4, 1989, Seiten 361 - 366
- PRATT ET AL.: 'Ig V Region Gene Expression in small Lymphocytic Lymphoma with Little or no Somatic Hypermutation' THE JOURNAL OF IMMUNOLOGY Bd. 143, Nr. 2, 15 Juli 1989, Seiten 699 - 705
- GILLIES ET AL.: 'Expression of Human anti-tetanus toxoid antibody in transfected murine myeloma cells' BIO/TECHNOLOGY Bd. 7, August 1989, Seiten 799 - 804
- Affidavit Amann of 19 March 2001
- BETTER ET AL.: 'Science', 30 Mai 1988 Seiten 1041 - 1043
- WEHLAND ET AL.: 'Amino acid sequence requirements in the epitope recognized' THE EMBO JOURNAL Bd. 3, Nr. 6, 1984, Seiten 1295 - 1300
- Brenner et al., 1989 FASEB J., 3:A1093
- IVERSON ET AL.: 'A combinatorial system for cloning and expressing the' COLD SPRING HARBOR SYMPOSIA ON QUANTITATIVE BIOLOGY 1989, Seiten 273 - 280
- GUSSOW ET AL.: 'Generating Binding Activities from Escherichia coli by Expression of a Repertoire of Immunoglobulin Variable Domains' COLD SPRING HARBOR SYMPOSIA ON QUANTITATIVE BIOLOGY 1989, Seiten 265 - 272
- Larrick et al., 1990 ICSU Short Report 10:93
- Front page and preface of ICSU Short Report 10
- FASEB J. Bd. 3, Oktober 1989, Seite 2433

## Beschreibung

Die Erfindung betrifft die Herstellung und Verwendung von Genbanken menschlicher Antikörper (AK). Ausgehend von einem Gemisch humaner B-Lymphozyten wird deren mRNA, unter Verwendung von Oligo-dT-Primern in cDNA übersetzt. Anschließend findet eine Amplifikation der AK-spezifischen cDNA mittels Polymerase-Ketten Reaktion (PCR, Polymerase Chain Reaction) unter Einsatz von geeigneten Oligonukleotid-Primersequenzen statt. Durch Expression dieser amplifizierten AK-spezifischen cDNA in einem bakteriellen Expressionsvektor, z.B. dem nachstehend beschriebenen Vektor pFMT, in E.coli, steht somit eine Human-Antikörper-Bibliothek mit einem umfassenden Repertoire zum Durchprüfen ("Screenen") mit ausgewählten Antigenen in vitro zur Verfügung.

Es wird geschätzt, daß das Immunsystem des Menschen bzw. eines Säugetiers zwischen 10⁶ und 10⁸ verschiedene Antikörper besitzt. Diese Zahl von Antikörpern reicht aus, um eine Immunreaktion des Körpers sowohl gegen sämtliche in der Natur vorkommende Antigene als auch gegen künstliche Antigene hervorzurufen. Wenn man weiterhin in Betracht zieht, daß oft mehrere Antikörper mit demselben Antigen reagieren, wird das Repertoire an wirklich verschiedenen Antikörpern eher im Bereich 10⁶ bis 10⁷ anzusiedeln sein.

Bisher wurden spezifische Antikörper stets ausgehend von einer Immunisierung mit dem jeweiligen Antigen erhalten, beispielsweise Injektion des Antigens in den Organismus oder Inkubation von Milzzellen in vitro mit diesem Antigen. Im Fall von polyklonalen Antikörpern lassen sich die Immunglobuline anschließend aus dem Serum isolieren und z.B. durch Absorptionsverfahren die spezifischen Antikörper daraus gewinnen. Monoklonale Antikörper werden aus den Zellüberständen bwz. dem Zell-Lysat von mit einzelnen B-Lymphozyten fusionierten, klonierten Milztumorzellen (Hybridomzellen) isoliert. Die oben geschilderten Verfahren sind insbesondere zur Herstellung von spezifischen Human-Antikörpern bzw. humanen monoklonalen Antikörpern nicht geeignet.

Die vorliegende Erfindung stellte sich deshalb die Aufgabe, eine allgemein gangbare Methode zur Erzeugung spezifischer humaner monoklonaler Antikörper (huMAK's) oder von Antikörperteilen, die die Antigenbindungsstelle enthalten, zu entwickeln.

Es wurde gefunden, daß die gesuchten huMAK's oder deren Teile, die die variable, antigenbindende Domäne enthalten, aus Genbanken humaner Immunglobline isoliert werden können. Zunächst wurde ausgehend von einem Gemisch nicht aktivierter humaner B-Lymphozyten deren mRNA isoliert und mit Hilfe von Oligo-dT-Primern in cDNA übersetzt. Eine spezifische Amplifizierung der Population von Antikörper cDNAs innerhalb des erhaltenen cDNA-Pools wurde durch die Verwendung der PCR erreicht. Dazu wurden bestimmte Oligonukleotid-"Primer" verwendet, die homolog zu konservierten Sequenzen an beiden Enden der Antikörper-cDNA sind (siehe unten und Beispiele). Der Entwurf der Primer für die Rückreaktion zur Synthese des nicht-kodierenden Stranges der DNA der schweren Kette basiert auf IgM-Sequenzen (Subklasse III, da diese die meisten IgM-Sequenzen umfaßt). IgM-Moleküle sind in nicht-aktivierten B-Lymphozyten häufiger vertreten als alle andern Klassen von Immunglobulinen. IgG-Sequenzen überwiegen dagegen in aktivierten B-Lymphozyten, deren Repertoire verschiedener Antikörper sehr viel kleiner ist. Bei einer IgG-Bibliothek bestände außerdem die Gefahr, daß ein oder wenige besonders stark exprimierte IgG die Bibliothek dominieren.

Bis zu 30 Amplifizierungs-Runden wurden zweckmäßigerweise durchgeführt. Die Oligonukleotid-"Primer" enthalten geeignete Restriktionsstellen, um die amplifizierte DNA z.B. in das Antikörper-Expressions-Plasmid pFMT (s.u.) zu insertieren. Dieses Expressions-Plasmid ermöglicht die Expression von Antikörper-cDNA und anschließende Sezernietung der Expressionsprodukte in Bakterien (E.coli). Das Antikörper-Operon des Plasmids enthält die Sequenzen der variablen Teile sowohl der schweren als auch der leichten Kette eines Antikörpers. Geeignete "Leader"-Sequenzen aus dem aminoterminalen Teil eines bakteriellen Proteins ermöglichen die Sezernierung der Antikörperteile. Die "Leader"-Sequenzen werden bei der Sekretion von einem bakteriellen Enzym abgespalten. Während der Sekretion der Antikörper-cDNA-Produkte assoziieren die leichten und schweren Ketten des Antikörpers (mit oder ohne angrenzende konstante Domäne). Dadurch wird ein Antikörper oder Antikörperfragment gebildet, der bzw. das eine funktionelle Antigen-Bindungsstelle enthält. Ähnliche Konstrukte für einzelne Antikörper sind auch von anderen Autoren beschrieben worden (Better et al. (1988), Science 240, 1041, und Skerras & Plückthun (1988), Science 240, 1038).

Die Amplifizierung von DNA, die für die variablen Teile von Antikörpern kodiert, wurde zwar von anderen Autoren beschrieben (Orlandi et al. (1989), Proc. Natl. Acad. Sci. 86, 3833; Sastry et al., (1989) Proc. Natl. Acad. Sci. 86, 5728; Ward et al. (1989), Nature 341, 544); Huse et al. (1989), Science 246, 275). Hier wurde jedoch die u.a. auch für Antikörper kodierende mRNA aus Hybridom-Zellen oder Milz-Lymphozyten nach Behandlung mit einem bestimmten Antigen isoliert. Deshalb wurden dort auch Primer-Sequenzen eingesetzt, die nur auf IgG-Sequenzen basieren. Das ist natürlich dort von Vorteil, wenn möglichst viele Antikörper-DNA-Klone gesucht werden, die von aktivierten Lymphozyten stammen. Mit Primern aus IgG-Sequenzen sind die Chancen viel größer, Klone zu finden, die DNA kodierend für Antikörper gegen das injizierte Antigen enthalten. Hinzuzufügen ist, daß in den vorstehenden Arbeiten murine und damit also nicht-humane Antikörper-DNA synthetisiert und zusätzlich unter Ausschluß von Bereichen der lambda-Kette amplifiziert wurde.

In der vorliegenden Erfindung kommen dagegen Primer-Sequenzen zum Einsatz, die homolog zu Sequenzen in den konstanten Domänen von IgM-cDNA sind. So kann die Erfindung am besten realisiert werden, d.h. eine sehr große Vielfalt an Antikörpern, nämlich das gesamte Antikörper-Repertoire, in Form einer Bibliothek zur Verfügung zu stellen. Die Expression in vorzugsweise E.coli ergibt dann die gesuchte Human-Antikörper-Bibliothek, in der die gesuchten humanen Antikörper bzw. Antikörperteile durch Screenen von bakteriellen Klonen mit dem Antigen der Wahl aufgefunden werden.

In Tab. 1 sind geeignete Oligonukleotidprimer zur Amplifikation zusammengestellt. Die Positionen der vorgenannten Primer auf der µ, kappa- und lambda-Kette sind schematisch in Tab. 2 dargestellt. Die molekularbiologischen Konstruktionen im einzelnen, darunter auch der Expressionsvektor, d.h. das Antikörper-Expressionsplasmid pFMT, sind in den nachfolgenden Beispielen beschrieben.

Die Erfindung betrifft folglich Human-Antikörper-Bibliotheken, hergestellt über Transkription der mRNA aus nicht-aktivierten (peripheren) humanen B-Lymphozyten mittels Oligo-dT-Primer, anschließende Amplifikation durch PCR unter Verwendung von Primern enthaltend Sequenzen homolog zu konservierten Bereichen von IgM-cDNA und nachfolgenden Einbau in geeignete Expressionsplasmide zur Expression in Mikroorganismen, vorzugsweise in den Expressionssvektor pFMT zur Expression in E.coli. In einer bevorzugten Ausführungsform wird zusätzlich eine Sequenz eingebaut, die für ein Markerpeptid kodiert, z.B. eine "TAG"-Sequenz, so daß die Expressionsprodukte mit etablierten monoklonalen Antikörpern gegen das Markerpeptid (Wehland et al., (1984), EMBO J. 3, 1295) auf einfache Weise nachgewiesen werden können.

Ferner umfaßt die Erfindung die Verwendung vorstehender Human-Antikörper-Bibliotheken zur Isolierung gesuchter humaner Antikörper bzw. Antikörperteile mit funktioneller Antigenbindungsstelle durch "Screenen" mit ausgewählten Antigenen sowie Verfahren zur Isolierung der genannten humanen Antikörper oder deren Antigen-bindenden Teile und auch Verfahren zur Herstellung der genannten Human-Antikörper-Bibliotheken.

Die nachfolgenden Beispiele führen die Erfindung weiter aus, ohne sie zu begrenzen. Schließlich ist die Erfindung in den Patentansprüchen enthalten.

### Beispiele:

### Beispiel 1: Herstellung eines Antikörper-Expressionsvektors

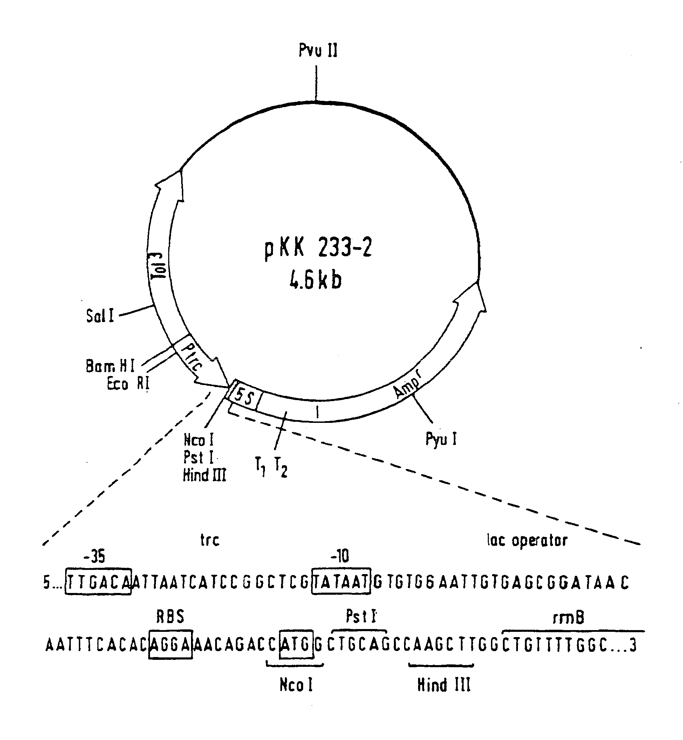

Das Plasmid pKK233-2 (Amann und Brosius, (1985) Gene 40, 183 und Straus und Gilbert, (1985) Proc. Natl. Acad. Sci. 82, 2014) wurde als Basisvektor für den Aufbau des Antikörper-Expressionsvektors gewählt (Fig. 1).

Vor dem Einbau des Antikörper-Operons wurde das Plasmid mit SalI und BamHI geschnitten, die Enden mit Klenow-Polymerase aufgefüllt und ligiert. Dadurch wurden diese beiden Restriktionsstellen und die dazwischenliegende DNA entfernt. Außerdem wurde das Plasmid mit HindIII gespalten, die Enden mit der Klenow-Polymerase aufgefüllt und mit BamHI-Linkern ligiert. Durch diese Operation wurde die HindIII-Restriktionsstelle entfernt und eine BamHI-Stelle insertiert. In dieses modifizierte Plasmid wurde die Antikörper-DNA insertiert. Ein schematischer Konstruktionsweg des Antikörper-Operons, das für eine bicistronische Antikörper-mRNA kodiert, wird in Tab. 3 gezeigt. Um die Sezernierung des Antikörpers zu ermöglichen, wurde die "Leader"-Sequenz des bakteriellen Enzyms Pektatlyase verwendet. Die "Leader"-Sequenz von diesem Enzym ist schon zur Expression und Sezernierung eines chimären Maus-Mensch-Antikörpers (Fab-Fragment, Better et al., a.a.O.) sowie des variablen Teils eines "humanisierten" Antikörpers (Ward et al., a.a.O.; Huse et al., a.a.O.) verwendet worden. DNA für die erste "Leader"Sequenz (P₁ vor der schweren Kette) und die Sequenz für eine zweite Ribosomenbindungsstelle (RBS) und eine zweite "Leader"-Sequenz (P₂ vor der leichten Kette) wurden aus mehreren Oligonukleotiden synthetisiert (Tab. 4).

Antikörper cDNAs, die für die variable Regionen der schweren und leichten Ketten eines menschlichen Antikörpers (HuVhlys bzw. HuVllys; Riechmann et al., (1988) J. Mol. Biol. 203, 825) kodieren, wurden von Dr. G. Winter (Cambridge, UK) erhalten. Die Restriktionsstellen HindIII (HuVhlys) und EcoRV (HuVllys) wurden eingeführt, um die Insertion der Antikörper-cDNA in den Expressionsvektor zu ermöglichen. Weitere Restriktionsstellen für BanII (HuVhlys) und BstEII bzw. KpnI (HuVllys) wurden eingeführt, um hypervariable Bereiche "en bloc" umzutauschen. Am Ende der HuVhlys-cDNA-Sequenz wurde ein Stopsignal eingebaut. Eine BanII-Stelle in der leichten Kette wurde entfernt. Diese Änderungen wurden mittels "site directed mutagenesis" in dem Bakteriophagen M13mp18 durchgeführt (Zoller und Smith, Meth. Enzymol. 100, 468-500). Die Sequenz der fertigen Antikörper-DNA ist in Tab. 5 gezeigt.

Für die Insertion der "Leader"-Sequenz P₁ (Tab. 4) wurde das modifizierte Plasmid pKK233-2 mit den Restriktionenzymen NcoI und PstI verdaut und P₁ zwischen diesen Stellen insertiert (pKK233-2-P₁). Weitere Klonierungsschritte bis auf den letzten Schritt wurden mit dem Plasmid pUC18 unternommen. Der Grund besteht darin, daß die Anwesenheit von einzelnen Teilen des Antikörper-Operons in dem Expressionsvektor das Wachstum des bakteriellen Wirts beeinträchtigt.

Vor der Klonierung in pUC18 mußte dessen BamHI-Restriktionsstelle entfernt-werden. Nach einem Verdau mit BamH1 wurden die einzelsträngigen Enden mit dem Klenow-Fragment aufgefüllt und religiert. Dieses modifizierte Plasmid wurde dann mit PstI und HindIII verdaut und P₂ plus RBS zwischen diesen Restriktionsstellen einligiert (pUC18-P₂). Bei dieser Operation verschwindet die ursprüngliche HindIII-Restriktionsstelle des Plasmids und eine neue HindIII-Restriktionsstelle wird eingebaut. pUC18-P₂ wurde dann mit PstI und HindIII verdaut und die DNA der schweren Kette (PstI-HindIII-Insert aus M13) wurde in diese beiden Stellen ligiert (pUC18-HP₂). Dieses Plasmid wurde dann mit EcoRV und BamHI verdaut und die DNA der leichten Kette (EcoRV-BamHI-Insert aus M13) wurde hineinligiert (pUC18-HP₂L).

In einer bevorzugten Ausführungsform wurde in die neue HindIII-Schnittstelle eine "TAG"-Sequenz hineinligiert (Tab. 4). Die "TAG"-Sequenz kodiert für das Peptid Glu-Glu-Gly-Glu-Glu-Phe und wird von dem monoklonalen Antikörper YL 1/2 (Wehland et al. (1984) EMBO J. 3, 1295) erkannt. Das resultierende Plasmid ist pUC-HTP₂L.

Für die Insertion von HP₂L bzw. HTP₂L in den Expressionsvektor wurden pUC18-HP₂L bzw. pUC-HTP₂L mit PstI und BamHI geschnitten und das betreffende Restriktionsfragment jeweils in das modifizierte Plasmid pKK233-2-P₁ in diese beiden Restriktionsstellen ligiert. Eine schematische Darstellung des fertigen Expressionsvektors pFMT wird in Tab. 6 gezeigt.

### Beispiel 2: Gewinnung von RNA aus menschlichen B-Lymphozyten

Zur Anreicherung peripherer B-Zellen aus menschlichem Blut wurde dieses mit PBS ("phosphate buffered saline") 1:1 verdünnt und über ein Ficoll^{R}(Pharmacia)-Kissen (1,077 kg/l) zentrifugiert. Die Zellen der Interphase wurden mit PBS zweimal gewaschen und in RPMI-Medium, das 10% fötales Kälberserum enthielt, auf einer Plastikunterlage (Kulturflasche) für eine Stunde bei 37°C inkubiert. Die adhärenten Zellen (Monocyten und Makrophagen) heften sich an das Kultur-Gefäß und konnten so aus der Präparation entfernt werden. Die nicht-adhärenten Zellen wurden durch Zentrifugation gesammelt und in 4,4 M Guanidiniumisothiocyanat, 5% Mercaptoethanol und 2% Lauroylsarcosin homogenisiert. Das Homogenat wurde dann über ein Kissen von 5,7 M CsCl für 18 Stunden bei 125000g zentrifugiert. Die sedimentierte RNA wurde in bidest. H₂O gelöst und mit 70% Ethanol und 1/20 Vol. 8M LiCl bei -20°C über Nacht gefällt.

Um eine noch größere Vielfalt von Antikörpern verschiedener Spezifität zu erhalten, wurden RNA-Präparationen von jeweils 500 ml des Blutes von 20 verschiedenen Personen gemischt.

### Beispiel 3: Amplifizierung der Antikörper-DNA

Die mRNA wurde über Oligo-dT-Sepharose (Kit von Pharmacia) gereinigt und mit reverser Transkriptase (Kit von Amersham) und Oligo-dT-Primer in cDNA übersetzt. Die Produkte wurden direkt in der "polymerase chain reaction" (PCR) verwendet. PCR-Primer und Hybridisierungsstellen werden in Tab. 1 bzw. 2 gezeigt. Durch eine Kombination der erhaltenen µ-DNA mit entweder kappa- oder lambda-DNA im Vektor pFMT wurden zwei verschiedene Expressionsbanken hergestellt. Die Verwendung verschiedener Primer für die Synthese der nicht-kodierenden Stränge in der "polymerase chain reaction" ermöglicht die Herstellung zweier verschiedener Antikörper-Typen, die in einem Fall nur die variable Domäne, im anderen Fall noch zusätzlich eine konstante Domäne enthalten (dem Fab-Fragment eines Antikörpers ähnlich). Für die PCR wurden 4 µl einer cDNA Synthese mit jeweils 0,2 nmol der zwei Primer in einem Volumen von 50 µl umgesetzt. Der Reaktionsansatz enthielt 100 mM KCl, 0,1% Gelatine und 2,5 U Taq-Polymerase. Nach 30 Polymerisationszyklen, bestehend aus 1 Min. 95°C, 2 Min. 55°C und 2 Min. 72°C wurde die DNA mit Ethanol gefällt.

### Beispiel 4: Insertion der Antikörper-DNA in das Expressionsplasmid

Die gefällte DNA wurde in Ladepuffer für Agarosegele (0.1% Bromphenolblau, 7% Ficoll^{R} [Pharmacia]) aufgenommen und über 2% Agarose bei10V/cm in TBE-Puffer (45 mM Tris-Borat pH 8.0, 10 mM EDTA) aufgetrennt. Die synthetisierte Antikörper-DNA wurde anhand ihres Molekulargewichtes identifiziert und aus dem Gel eluiert. Nach Fällung mit Ethanol wurde sie in Puffer für die jeweiligen Restriktionsenzyme aufgenommen und mit den entsprechenden (vgl. Tab. 1 und 2) Restriktionsenzymen (Boehringer Mannheim) geschnitten . Nach einer Fällung in Ethanol wurde sie in den ebenso geschnittenen Vektor pFMT einligiert, wie in der Tab. 7 schematisch gezeigt.

### Beispiel 5: Expression und "Screenen" von Antikörpern in E.coli

Kompetente E.coli werden mit pFMT-Plasmiden, die die insertierte Antikörper-DNA-Bibliothek enthalten, transfiziert, auf Agaroseplatten aufgezogen und anschließend mit Nitrozellulosefiltern inkubiert, die mit dem gesuchten Antigen beschichtet sind. Nach der Entfernung unspezifisch gebundener Antikörper werden die aktiven Klone mit einem markierten Antikörper gegen die aus E.coli sezernierten menschlichen Immunglobuline identifiziert. In der bevorzugten Ausführungsform wird zur Identifizierung der gesuchten Klone der monoklonale Antikörper YL 1/2 verwendet, der gegen die "TAG"-Sequenz gerichtet ist.

### Legende zu Fig. 1:

Restriktionskarte des Expressionsvektors pKK233-2 (Amann und Brosius, a.a.O.).
Ptrc bedeutet hybrider Tryptophan-lac Promotor
RBS bedeutet Ribosomenbindungsstelle
rrnB bedeutet ribosomale RNA B (5S RNA)
5S bedeutet Gen für 5S RNA (enthält rrnB)

Vor der Klonierung von Antikörper-DNA in den Expressionsvektor wurden die folgenden Änderungen durchgeführt:
1) Die SalI und EcoRI Restriktionsstellen wurden zusammen mit der zwischenliegenden DNA entfernt.
2) Die HindIII Restriktionsstelle wurde in eine BamHI Restriktionsstelle umgewandelt.

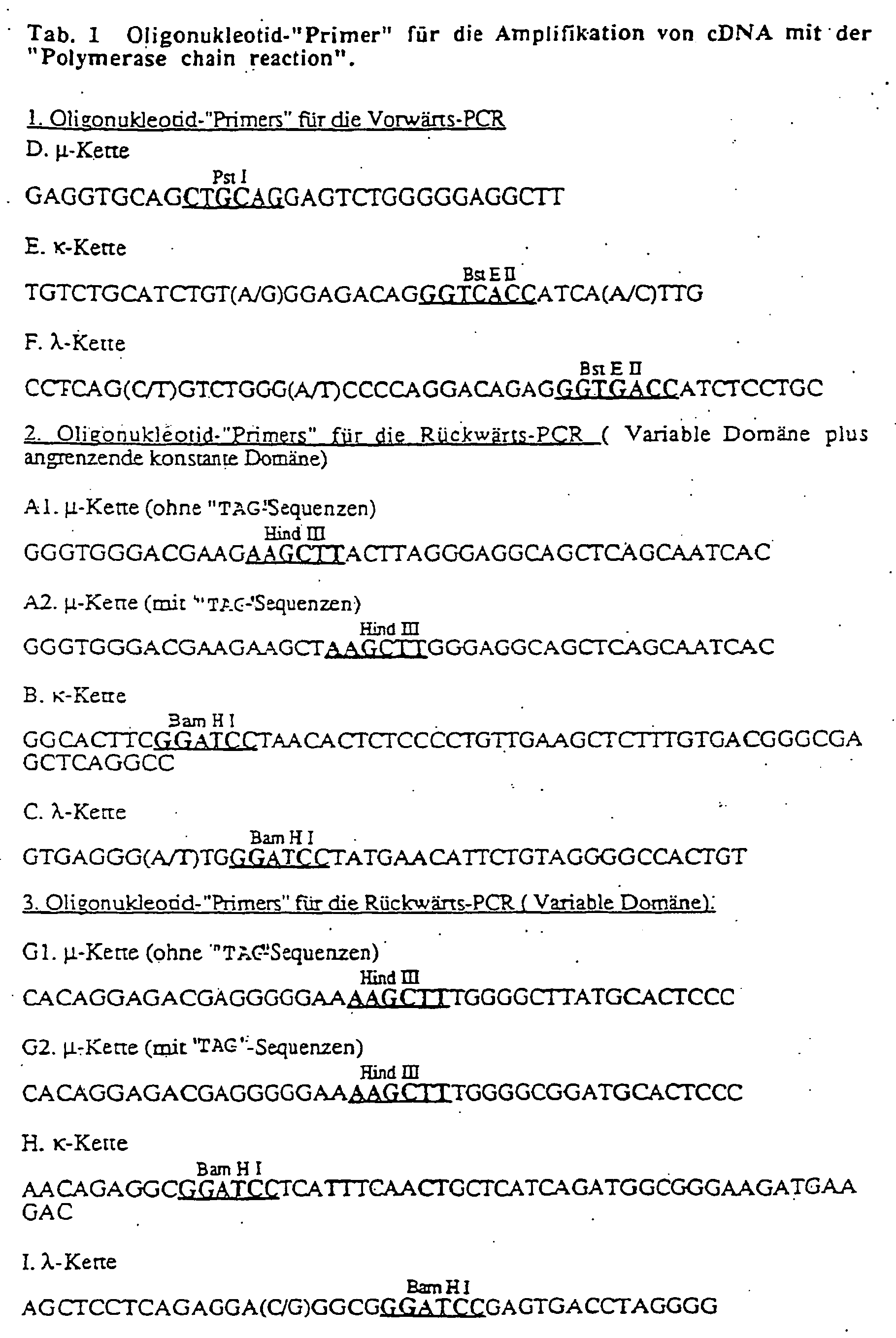

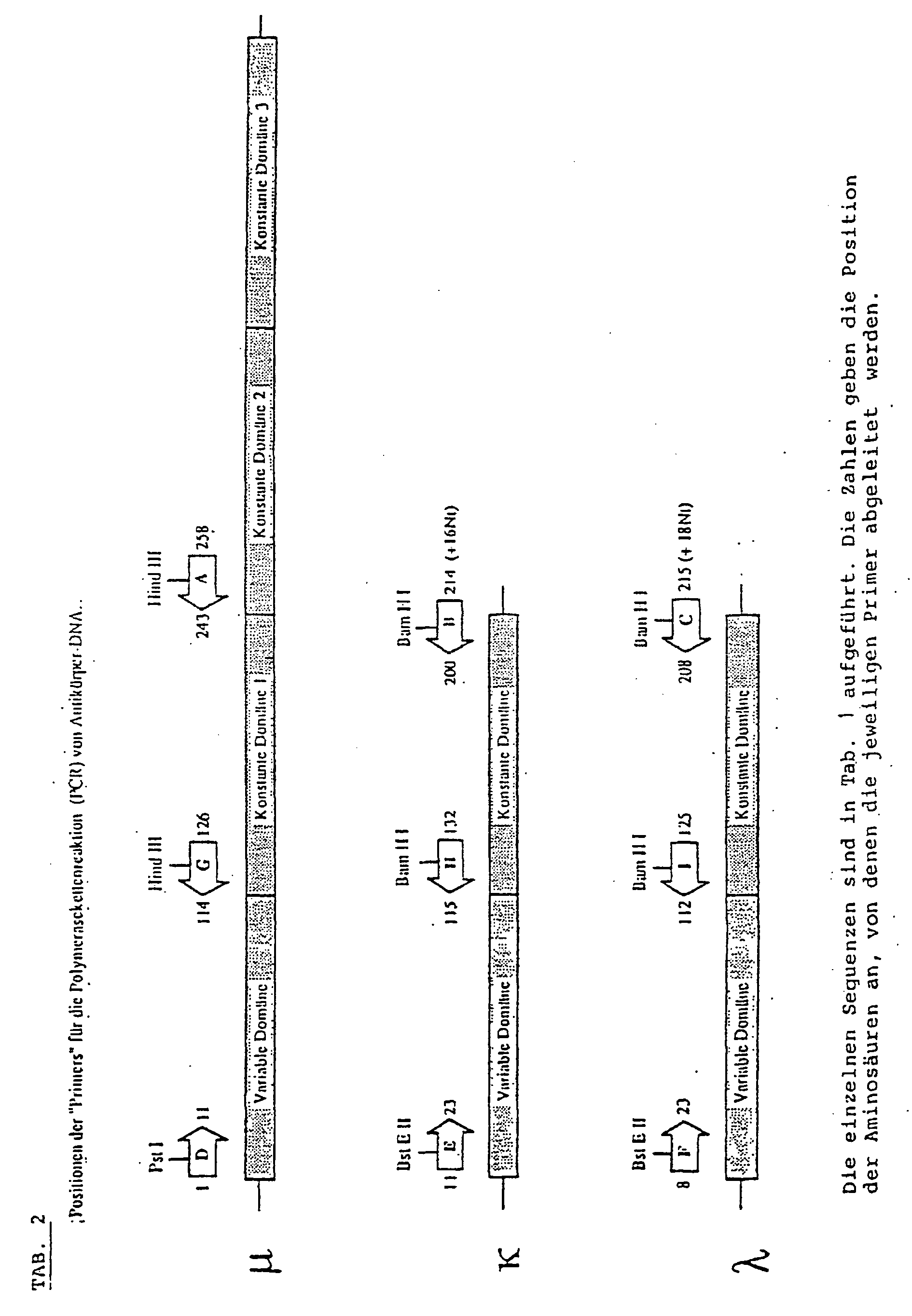

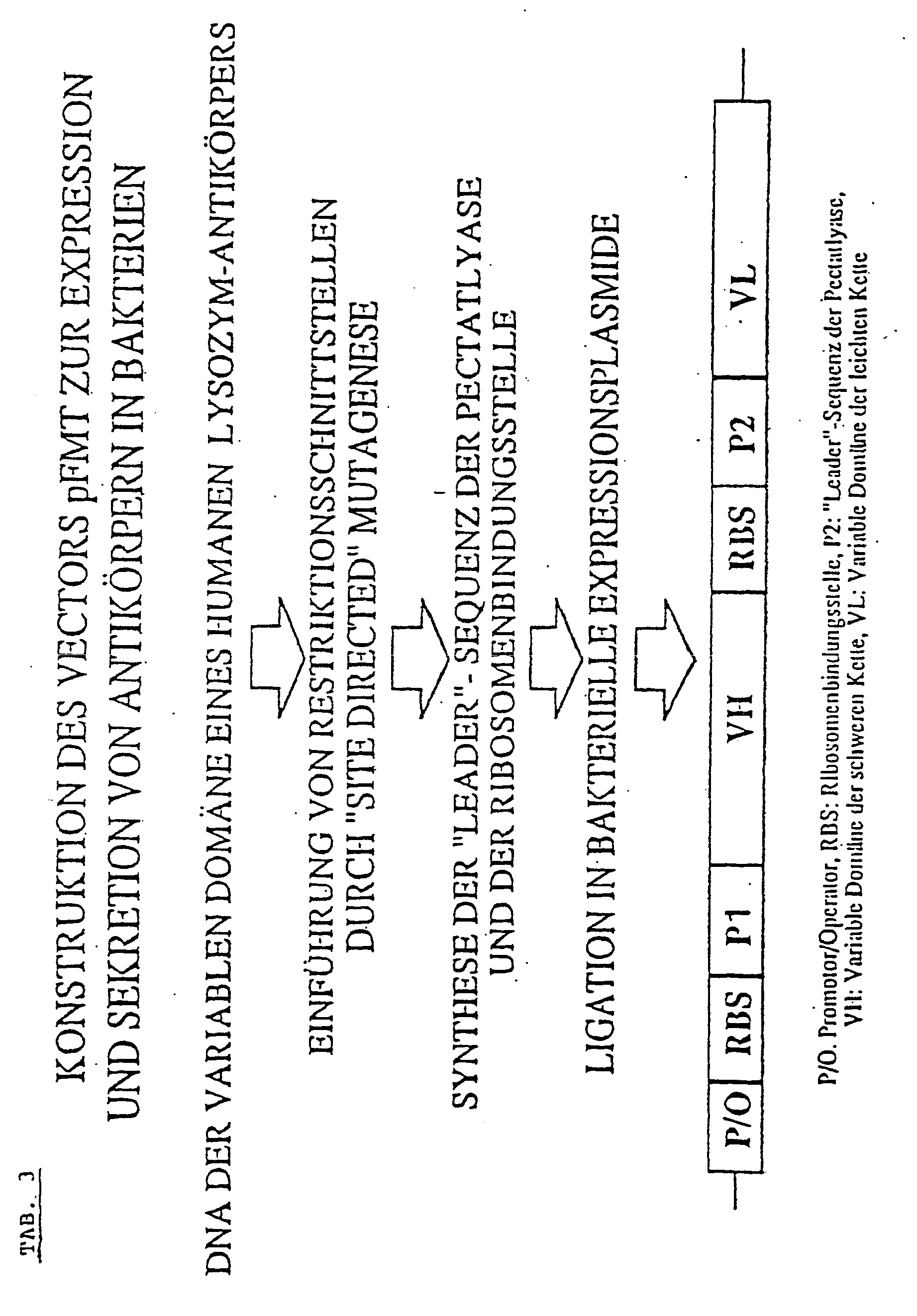

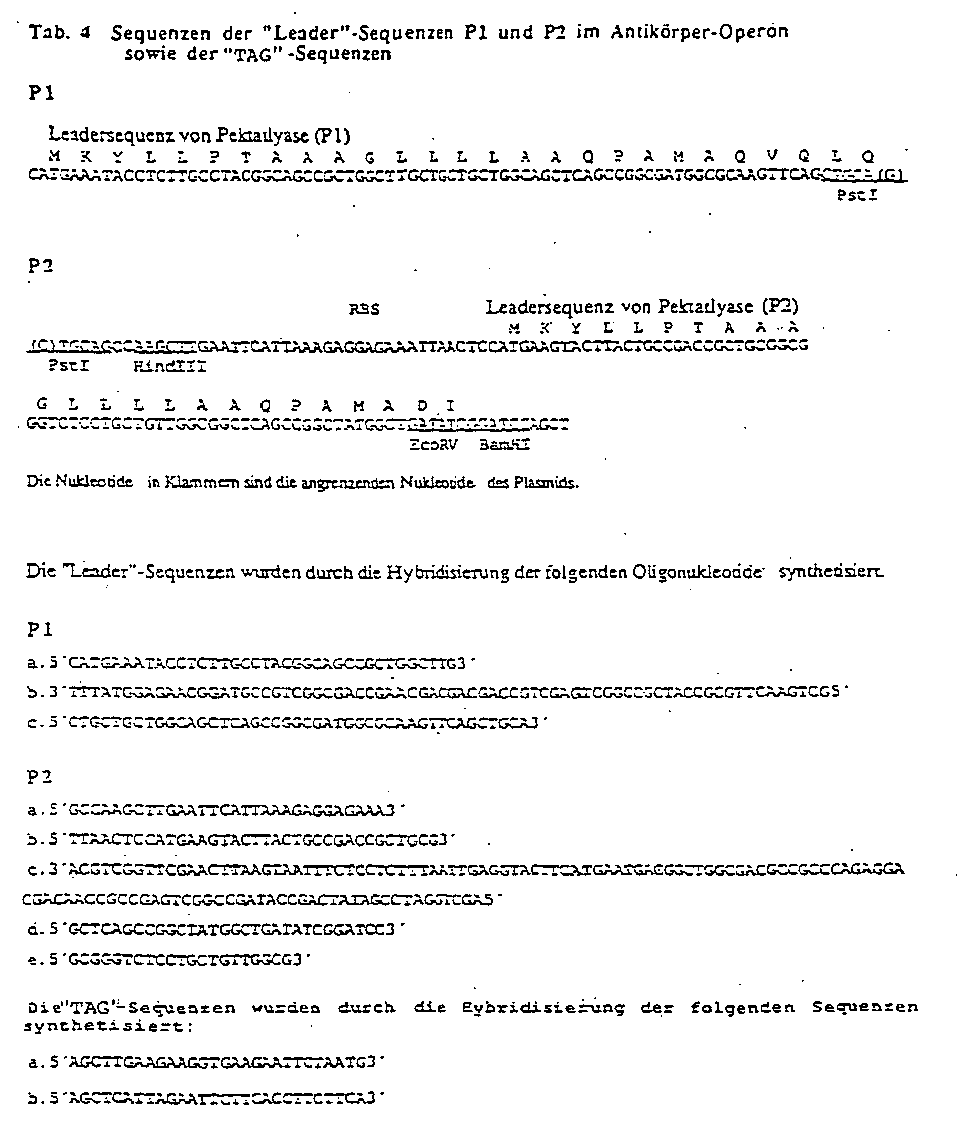

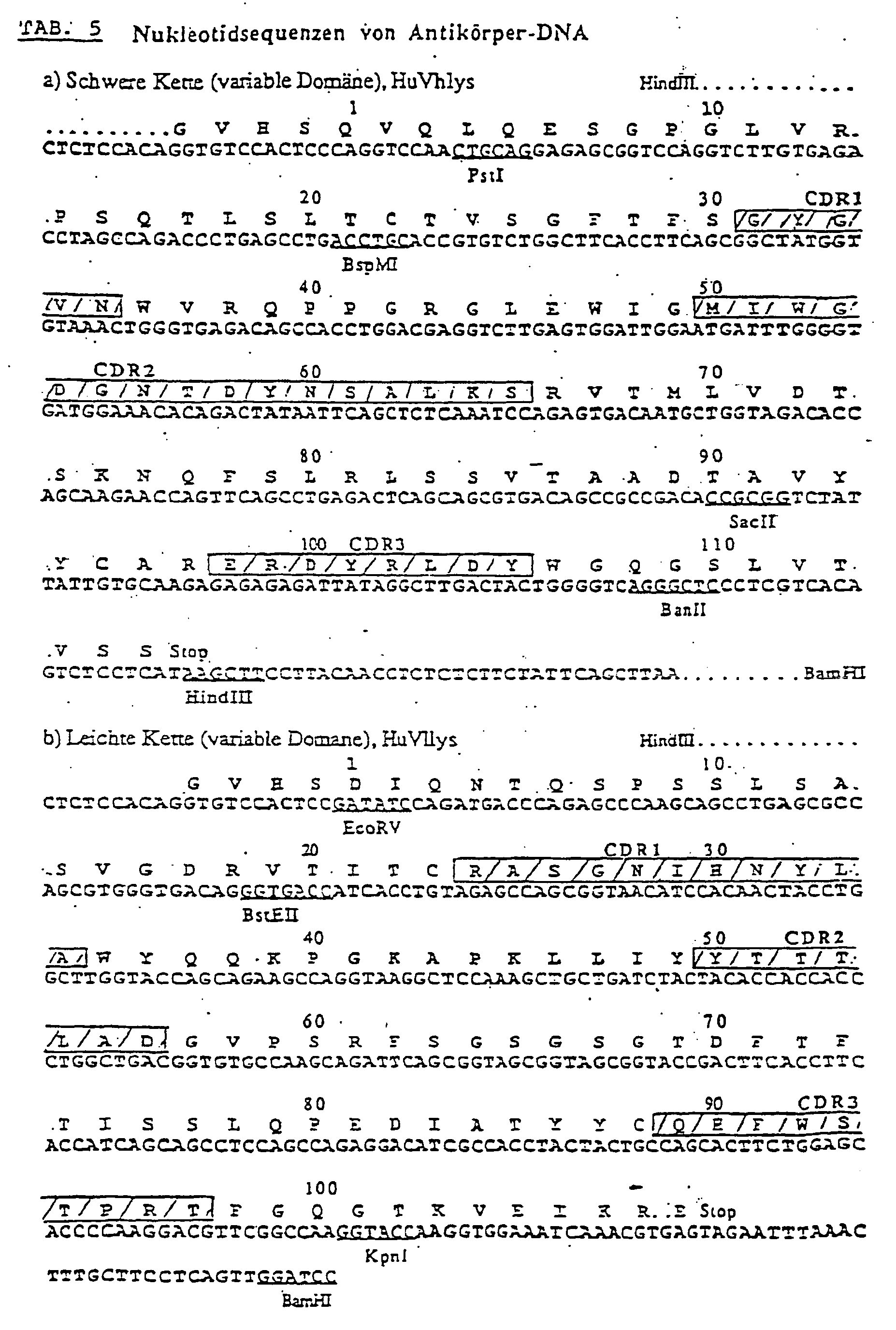

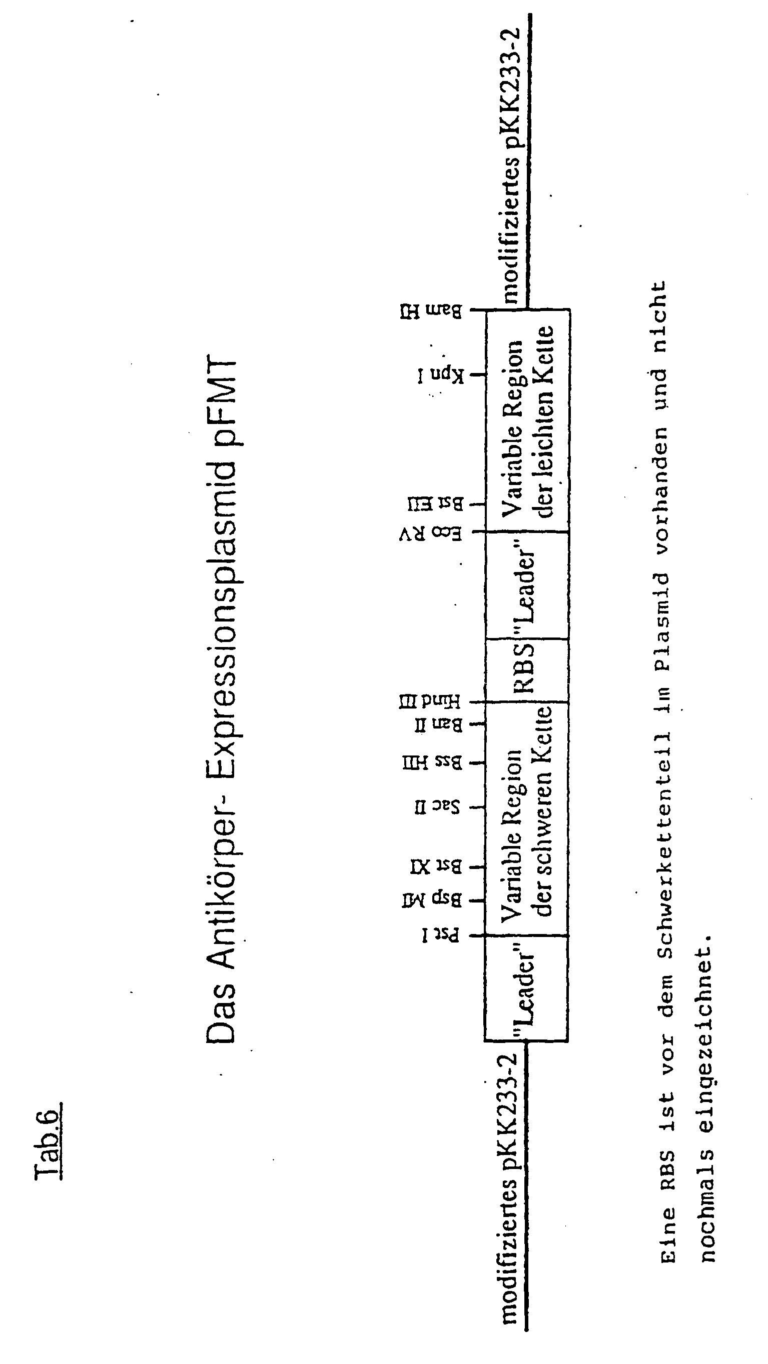

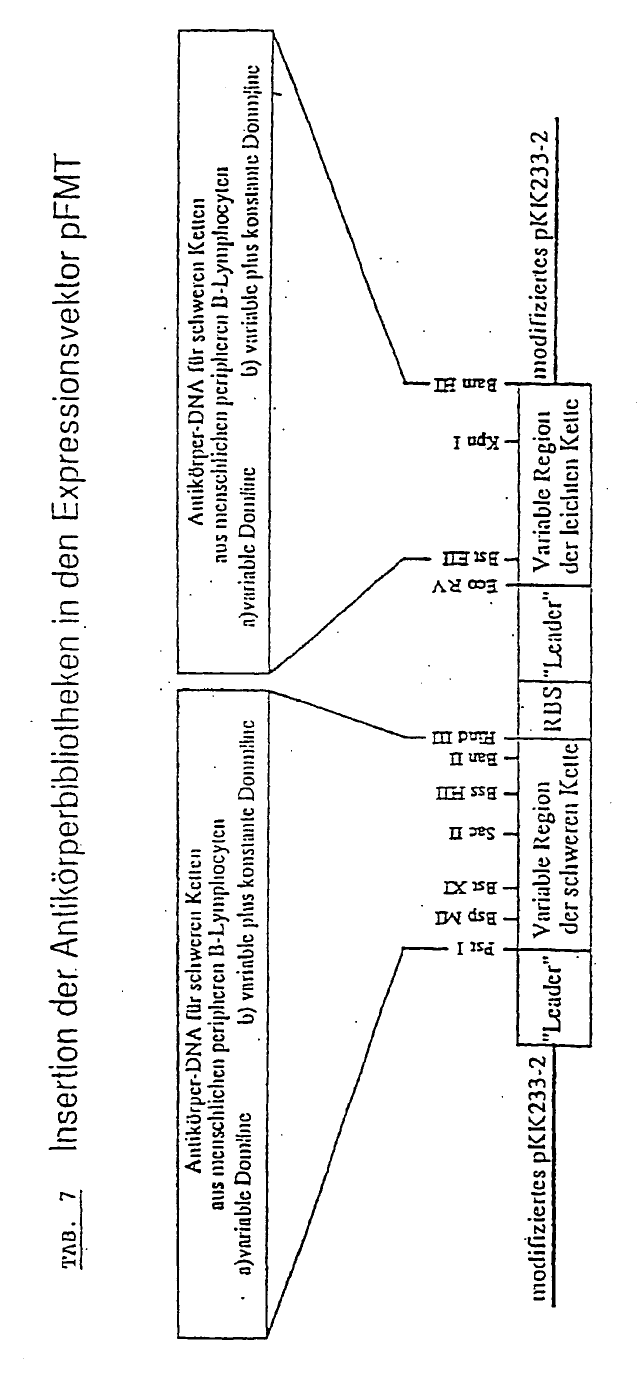

## Patentansprüche (Patentansprüche für folgende(n) Vertragsstaat(en): AT, BE, CH, LI, DE, DK, FR, GB, IT, LU, NL, SE)

1. Human-Antikörper-Bibliotheken, erhältlich über Transskription isolierter mRNA aus nicht aktivierten peripheren humanen B-Lymphozyten in cDNA, anschließende Amplifikation von für Antikörper kodierender cDNA durch "Polymerase Chain Reaction" (PCR) mittels geeigneter Primer und nachfolgenden Einbau in geeignete Expressionsplasmide und schließlich Expression der darin enthaltenen, in cDNA amplifizierten relevanten Antikörper-RNA in Einzelklonen, **dadurch gekennzeichnet, daß** durch die verwendeten Primer jeweils nur die variablen Regionen amplifiziert werden und die Expressionsplasmide für keine konstante Domäne eines Antikörpers kodieren, und der Entwurf der Primer für die Rückreaktion zur Synthese des nicht-kodierenden Stranges der DNA der schweren Kette auf IgM-Sequenzen basiert.

2. Human-Antikörper-Bibliotheken nach Anspruch 1, **dadurch gekennzeichnet, daß** die Expression im Plasmid pFMT gemäß Tab. 6 erfolgt.

3. Human-Antikörper-Bibliotheken nach Anspruch 1 oder 2, **dadurch gekennzeichnet, daß** IgM-spezifische Primer im PCR-Schritt eingesetzt werden.

4. Verfahren zur Herstellung von Human-Antikörper-Bibliotheken, **dadurch gekennzeichnet, daß** mRNA aus nicht aktivierten peripheren humanen B-Lymphozyten isoliert und in cDNA überschrieben wird, anschließend die für Antikörper kodierende cDNA durch PCR mittels geeigneter Primer amplifiziert wird, darauf folgend ein Einbau in geeignete Expressionsplasmide vorgenommen wird und schließlich Expression der Antikörper-cDNA in Einzelklonen erfolgt und daß durch die verwendeten Primer jeweils nur die variablen Regionen amplifiziert werden, die Expressionsplasmide für keine konstante Domäne eines Antikörpers kodieren und daß der Entwurf der für die Rückreaktion zur Synthese des nicht-kodierenden Stranges der DNA der schweren Kette verwendeten Primer auf IgM-Sequenzen basiert.

5. Verfahren nach Anspruch 4, **dadurch gekennzeichnet, daß** die Expression im Plasmid pFMT gemäß Tab. 6 erfolgt.

6. Verfahren zur Isolierung von spezifischen humanen Antikörpern, **dadurch gekennzeichnet, daß** Human-Antikörper-Bibliotheken nach Anspruch 1, 2 oder 3 mit spezifischen Antigenen durchsucht werden.

7. Verwendung von Human-Antikörper-Bibliotheken nach Anspruch 1, 2 oder 3 zur Isolierung von spezifischen humanen Antikörpern.

## Patentansprüche (Patentansprüche für folgende(n) Vertragsstaat(en): ES)

1. Verfahren zur Herstellung von Human-Antikörper-Bibliotheken, **dadurch gekennzeichnet, daß** mRNA aus nicht aktivierten peripheren humanen B-Lymphozyten isoliert und in cDNA überschrieben wird, anschließend die für die Antikörper kodierende cDNA durch PCR mittels geeigneter Primer amplifiziert wird, darauf folgend ein Einbau in geeignete Expressionsplasmide vorgenommen wird und schließlich Expression der Antikörper-cDNA in Einzelklonen erfolgt und daß durch die verwendeten Primer jeweils nur die variablen Regionen amplifiziert werden, die Expressionsplasmide für keine konstante Domäne eines Antikörpers kodieren und daß der Entwurf der für die Rückreaktion zur Synthese des nicht-kodierenden Stranges der schweren Kette verwendeten Primer auf IgM-Sequenzen basiert.

2. Verfahren nach Anspruch 1, **dadurch gekennzeichnet, daß** die Expression im Plasmid pFMT gemäß Tab. 6 erfolgt.

3. Verfahren zur Isolierung von spezifischen humanen Antikörpern, **dadurch gekennzeichnet, daß** Human-Antikörper-Bibliotheken hergestellt nach Anspruch 1 oder 2 mit spezifischen Antigenen durchsucht werden.

## Claims (Claims for the following Contracting State(s): AT, BE, CH, LI, DE, DK, FR, GB, IT, LU, NL, SE)

1. A human-antibody library obtainable by means of transcription of isolated mRNA from nonactivated peripheral human B-lymphocytes into cDNA, subsequent amplification of cDNA coding for antibodies by polymerase chain reaction (PCR) by means of suitable primers, and subsequent' incorporation into suitable expression plasmids and finally expression, in individual clones, of the relevant antibody RNA which is contained therein and has been amplified in cDNAs, wherein only the variable regions are amplified in each case by the primers used and the expression plasmids do not code for any constant domains of an antibody; and the design of the primer for the reverse reaction for the synthesis of the noncoding strand of the DNA of the heavy chains is based on IgM sequences.

2. A human-antibody library as claimed in claim 1, wherein the expression takes place in the plasmid pFMT according to Tab. 6.

3. A human-antibody library as claimed in claim 1 or 2, wherein IgM-specific primers are used in the PCR step.

4. A process for preparing a human-antibody library, which comprises mRNA being isolated from nonactivated peripheral human B-lymphocytes and being transcribed into cDNA, subsequently amplifying the cDNA coding for antibodies by PCR by means of suitable primers, then carrying out an incorporation into suitable expression plasmids and finally expressing the antibody cDNA in individual clones, and wherein only the variable regions are amplified in each case by the primers used, the expression plasmids do not code for any constant domains of an antibody, and wherein the design of the primer used for the reverse reaction for the synthesis of the noncoding strand of the DNA of the heavy chains is based on IgM sequences.

5. The process as claimed in claim 4, wherein the expression takes place in plasmid pFMT according to Tab. 6.

6. A process for isolating specific human antibodies, comprising screening human-antibody libraries as claimed in claim 1, 2 or 3 using specific antigens.

7. The use of a human-antibody library as claimed in claim 1, 2 or 3 for isolating specific human antibodies.

## Claims (Claims for the following Contracting State(s): ES)

1. A process for preparing a human-antibody library, which comprises mRNA being isolated from nonactivated peripheral human B-lymphocytes and being transcribed into cDNA, subsequently amplifying the cDNA coding for antibodies by PCR by means of suitable primers, then carrying out an incorporation into suitable expression plasmids and finally expressing the antibody cDNA in individual clones and wherein only the variable regions are amplified in each case by the primers used, the expression plasmids do not code for any constant domains of an antibody, and wherein the design of the primer used for the reverse reaction for the synthesis of the noncoding strand of the heavy chains is based of IgM sequences.

2. The process as claimed in claim 1, wherein the expression takes place in plasmid pFMT according to Tab. 6.

3. A process for isolating specific human antibodies, comprising screening human-antibody libraries prepared as claimed in claim 1 or 2 using specific antigens.

## Revendications (Revendications pour l'(les) Etat(s) contractant(s) suivant(s): AT, BE, CH, LI, DE, DK, FR, GB, IT, LU, NL, SE)

1. Bibliothèques d'anticorps humains, disponibles via la transcription d'ARNm isolés à partir de lymphocytes B humains périphériques non activés, en ADNc, l'amplification subséquente de l'ADNc codant pour les anticorps par réaction en chaîne par polymérase (PCR) au moyen d'une amorce appropriée et la construction consécutive dans des plasmides d'expression appropriés et enfin, l'expression des ARN des anticorps appropriés, contenus à l'intérieur, amplifiés en ADNc en clones uniques, **caractérisées en ce que**, par les amorces utilisées, respectivement seules les régions variables sont amplifiées, et les plasmides d'expression ne codent pour aucun domaine constant d'un anticorps est le modèle d'amorce utilisé pour la réaction de retour pour la synthèse du brin non codant de l'ADN de la chaîne lourde est basé sur des séquences d'IgM.

2. Bibliothèques d'anticorps humains selon la revendication 1, **caractérisées en ce que** l'expression se produit dans le plasmide pFMT selon le tableau 6.

3. Bibliothèques d'anticorps humains selon la revendication 1 ou 2, **caractérisées en ce que** les amorces spécifiques d'IgM sont insérées dans l'étape de PCR.

4. Procédé de fabrication de bibliothèques d'anticorps humains, **caractérisé en ce que** l'ARNm est isolé à partir de lymphocytes B humains périphériques non activés et est transcrit en ADNc, ensuite l'ADNc codant pour les anticorps est amplifié par PCR au moyen d'une amorce appropriée, dont il s'ensuit la mise en oeuvre d'une construction dans un plasmide d'expression approprié et enfin il se produit l'expression des ADNc d'anticorps en clones uniques, et **en ce que**, par les amorces utilisées respectivement, seules les régions variables sont amplifiées, les plasmides d'expression ne codent pour aucun domaine constant d'un anticorps et que le modèle d'amorces utilisé pour la réaction de retour pour la synthèse du brin non codant de l'ADN de la chaîne lourde est basé sur des séquences d'IgM.

5. Procédé selon la revendication 4, **caractérisé en ce que** l'expression se produit dans le plasmide pFMT selon le tableau 6.

6. Procédé d'isolement des anticorps humains spécifiques, **caractérisé en ce que** des bibliothèques d'anticorps humains selon la revendication 1, 2 ou 3 sont criblées avec des antigènes spécifiques.

7. Utilisation de bibliothèques d'anticorps humains selon la revendication 1, 2 ou 3 pour l'isolement des anticorps humains spécifiques.

## Revendications (Revendications pour l'(les) Etat(s) contractant(s) suivant(s): ES)

1. Procédé de fabrication de bibliothèques d'anticorps humains, **caractérisé en ce que** l'ARNm est isolé à partir de lymphocytes B humains périphériques non activés et est transcrit en ADNc, ensuite l'ADNc codant pour les anticorps est amplifié par PCR au moyen d'une amorce appropriée, dont il s'ensuit la mise en oeuvre d'une construction dans un plasmide d'expression approprié et enfin il se produit l'expression des ADNc d'anticorps en clones uniques, et **en ce que**, par les amorces utilisées respectivement, seules les régions variables sont amplifiées, les plasmides d'expression ne codent pour aucun domaine constant d'un anticorps et que le modèle d'amorces utilisé pour la réaction de retour pour la synthèse du brin non codant de l'ADN de la chaîne lourde est basé sur des séquences d'IgM.

2. Procédé selon la revendication 1, **caractérisé en ce que** l'expression se produit dans le plasmide pFMT selon le tableau 6.

3. Procédé d'isolement des anticorps humains spécifiques, **caractérisé en ce que** des bibliothèques d'anticorps humains selon la revendication 1 ou 2, sont criblées avec des antigènes spécifiques.
